# EUROPÄISCHE PATENTSCHRIFT

(11) **EP 1 928 395 B1**
(45) Veröffentlichungstag und Bekanntmachung des Hinweises auf die Patenterteilung: **11.03.2015**
(21) Anmeldenummer: 05805165.7
(22) Anmeldetag: 22.09.2005
(51) Int. Cl.: A61K 6/083, A61K 6/027

(54) **DENTALMATERIAL**
DENTAL MATERIAL
MATERIAU DENTAIRE

(43) Veröffentlichungstag der Anmeldung: 11.06.2008
(73) Patentinhaber: GDF GESELLSCHAFT FÜR DENTALE FORSCHUNG UND INNOVAT, D-61191 Rosbach v.d.H. (DE); Micerium SPA, 16030 Avegno GE (IT); Vanini, Lorenzo, 22028 San Fedele Intelvi (CO) (IT)
(72) Erfinder: VANINI, Lorenzo, I-22028 San Fedele Intelvi (IT); MICELI, Eugenio, I-16145 Genova (IT); NIEM, Thomas, 34497 Korbach (DE)
(74) Vertreter: Lusuardi, Werther
(86) Internationale Anmeldenummer: PCT/IB2005/002816
(87) Internationale Veröffentlichungsnummer: WO 2007/034258

(56) Entgegenhaltungen:
- EP-A- 0 710 475
- EP-A- 1 149 573
- WO-A-00/21488
- WO-A-01/26611
- DE-A1- 19 741 286

## Beschreibung

Die Erfindung bezieht sich auf ein Dentalmaterial gemäss dem Oberbegriff des Patentanspruchs 1.

Die bekannten Dentalmaterialien, insbesondere Füllungsmaterialien und Dentallacke sind aus verschiedenartigen flüssigen und festen Substanzen zusammengesetzt, welche in der Regel alle unterschiedliche Brechungsindizes aufweisen. Aber auch insgesamt weisen die bekannten Dentalmaterialien Brechungsindizes auf, welche nicht demjenigen des natürlichen Zahnes entsprechen. Dies führt zu einem ungünstigen optischen Verhalten der Dentalmaterialien, wenn sie im oder am Zahn aufgebracht werden. Während der natürliche Zahnschmelz mit zunehmender Schichtdicke, weisslicher und leuchtender wird und damit den Grauwert reduziert, verhalten sich die bisher bekannten Dentalmaterialien gerade umgekehrt, indem ihr Grauwert mit zunehmender Schichtdicke zunimmt (Glaseffekt). Die optischen Eigenschaften der mit solchen bekannten Materialien erhaltenen Restaurationen entsprechen somit nicht denen des natürlichen Schmelzes. Aus der EP-A 0 710 475 ist ein Dentalmaterial bekannt, welches auf einem Methacrylatmonomer mit hohem Brechungsindex basiert. Aus der WO 01/26611 ist ein Dentalmaterial bekannt, welches opaleszierende Eigenschaften aufweist.

Es besteht deshalb ein Bedürfnis ein Dentalmaterial, insbesondere zur Rekonstruktion von Schmelzpartien zu schaffen, welches auch in grösseren, dem natürlichen Schmelz entsprechenden Schichtdicken auf den zu restaurierenden Zahn aufgebracht werden kann.

Hier will die Erfindung Abhilfe schaffen. Der Erfindung liegt die Aufgabe zugrunde, ein Dentalmaterial zu schaffen, welches dem zu restaurierenden Zahn optimale optische Eigenschaften verleiht und auch in dicken Schichtdicken aufbringbar ist.

Die Erfindung löst die gestellte Aufgabe mit einem Dentalmaterial, welches die Merkmale des Anspruchs 1 aufweist.

Die durch die Erfindung erreichten Vorteile sind im wesentlichen darin zu sehen, dass:
A) das erfindungsgemässe Dentalmaterial - insbesondere wenn es als Schmelzschicht verwendet wird - auch mit grösserer Schichtdicke aufgetragen werden kann und damit einen schmelzähnlichen, optischen Effekt erzielen kann;
B) das erfindungsgemässe Dentalmaterial in jeglicher Weise bezüglich der Lichtbrechung dem natürlichen Schmelz entspricht, insbesondere weist es eine optimale Transparenz und Tiefenwirkung auf;
C) durch die Möglichkeit dickere Schichten aufzutragen das erfindungsgemässe Material ein einfacheres und besseres Handling des Materials gestattet;
D) dank der dickeren Schichtdicke sich auch eine bessere Resistenz des Materials gegenüber chemischen Angriffen im Mundmilieu ergibt.

Bei einer besonderen Ausführungsform der Erfindung besitzt das Dentalmaterial einen Brechungsindex n₄ grösser als 1,59, vorzugsweise grösser als 1,60. Der Brechungsindex n₄ des Dentalmaterials ist vorteilhafterweise kleiner als 1,64 vorzugsweise kleiner als 1,62.

Bei einer anderen Ausführungsform weist das allein mit den nanoskaligen anorganische Feststoffteilchen vermischte Dentalharz einen Brechungsindex n₁₂ auf, welcher um höchstens 5 %, vorzugsweise höchstens 2 % vom Brechungsindex n₃ der Dentalglaspartikel abweicht. Die in einem ersten Schritt in das Dentalharz eingemischten nanoskaligen anorganischen Feststoffteilchen führen zu einem klaren, transparenten, d.h. nicht opaken, kolloidalen System; durch die Auswahl eines Dentalglases mit annähernd gleichem Brechungsindex, werden die optischen Eigenschaften des Gesamtsystems weitgehend erhalten, das heisst auch das flüssige oder pastöse Dentalmaterial bleibt insgesamt transparent, was für dentale Anwendungen von grösster Bedeutung ist.

Der Brechungsindex n₁₂ sollte vorteilhafterweise höchstens um 1,0 %, vorzugsweise höchstens um 0,2 % vom Brechungsindex n₃ abweichen und im Bereich von 1,56 bis 1,65, vorzugsweise von 1,59 bis 1,62 liegen.

Der Brechungsindex n₃ der Dentalglaspartikel sollte vorteilhafterweise im Bereich von 1,58 bis 1,65, vorzugsweise von 1,59 bis 1,62 liegen. Der Brechungsindex n₁ des Dentalharzes ist vorteilhafterweise grösser als 1,54.

Als Dentalharze haben sich die Bis-Methacrylate, vorzugsweise Bis-GMA oder ein ethoxyliertes Bisphenol-A-dimethacrylat oder Derivate davon als geeignet für die Erfindung erwiesen. Das Dentalharz kann auch ein methacryl-substituiertes Poly-Di-Phenyl-silikon oder Diphenylsilan-Derivat sind. Ebenfalls geeignet sind Dentalharze aus der Gruppe: Poly(pentabromophenyl-methacrylate, Poly(pentabromophenylacrylate), Poly(pentabromobenzyl-methacrylate), Poly(pentabromobenzyl-acrylate, Poly(2,4,6-tribromophenyl-methacrylate), Poly(vinylphenylsulfide), Poly(1-naphthylmethacrylate), Poly(2-vinylthiophene, Poly(2,6-dichlorostyrene), Poly(N-vinylphthalimide), Poly(2-chlorostyrene), Poly(pentachlorophenyl methacrylate).

Die nanoskaligen anorganischen Feststoffteilchen sind vorteilhafterweise auf der Basis von Metalloxiden vorzugsweise Titandioxid, Zinkoxid, Zirkonoxid oder Aluminiumoxid, sowie Mischungen davon ausgewählt. Typischerweise werden Teilchen mit einem Durchmesser vorzugsweise zwischen 20 - 40 nm verwendet.

Bei einer besonderen Ausführungsform liegt das Gewichtsverhältnis zwischen dem Dentalharz und den nanoskaligen anorganischen Feststoffteilchen im Bereich von 1,0 bis 2,5.

Die Oberfläche der nanoskaligen anorganischen Feststoffteilchen kann mit einer organischen Säure, vorzugsweise einem Karbonsäurederivat oder einem methacrylatsubstituierten Silan beschichtet sein. Diese Beschichtung verhindert eine Reagglomerierung der nanoskaligen anorganischen Feststoffteilchen im Dentalharz. Überraschenderweise bleibt dieser nützliche Effekt auch noch nach Zugabe der Dentalglaspartikel zum kolloidalen System Dentalharz/Nanopartikel bestehen. Speziell geeignet sind die: 2-[2-(2Methoxyethoxy)-ethoxy]-essigsäure oder das Gammamethacryloxypropyltrimethoxysilan.

Die Dentalglaspartikel weisen vorteilhafterweise einen maximalen mittleren Durchmesser von 5 µm, vorzugsweise maximal 0,7 µm auf.

Als Dentalgläser eignen sich insbesondere Borosilkatgläser, Bariumaluminosilicatgläser, Silica, Titansilikat, Zirkoniumsilikat, Bariummagnesiumaluminosilikatgläser, Bariumoxid, Quartz und Aliminiumoxid. Die Dentalgläser können auch Gamma-methacryloxypropyltrimethoxysilanmodifiziert sein.

Als Dentalgläser eignen sich auch amorphe, kugelförmige Materialien auf der Basis von Mischoxiden aus SiO₂, ZrO₂, ZnO, La₂O₃, Al₂O₃ und/oder TiO₂ mit einer mittleren durchschnittlichen Partikelgrösse von 0,005 bis 5.0 µm, vorzugsweise von 0,1 bis 1µm, sowie Makro- und Mini-Füllstoffe wie Quarz-, Glaskeramik- oder Glaspulver, Bariumsilikatgläser, Bariumfluorsilikatgläser und Li/Al Silikatgläser, Bariumgläser, die Oxide von Aluminium, Zink, Lanthan, Zirkonium oder Silicium, mit einer durchschnittlichen Teilchengrösse von 0,01 bis 20µm, vorzugsweise 0,5 bis 5µm. Unter Mini-Füllstoffen werden Füllstoffe mit einer Partikelgrösse von 0, 5 bis 1,5µm und unter Makro-Füllstoffen Füllstoffe mit einer Partikelgrösse von 10 bis 20µm verstanden.

Weitere geeignete Dentalgläser sind Mischungen aus (A) amorphen, kugelförmigen Teilchen aus Siliciumdioxid und bis zu 20 Mol- % eines Oxids mindestens eines Elementes der Gruppen I, II, IV, V, XII, XIII und XIV des Periodensystems mit einem Brechungsindex von 1,50 bis 3,00 und mit einer durchschnittlichen Primärteilchengrösse von 0,1 bis 5,0 µm, und (B) Quarz-, Glaskeramik- oder Glaspulver oder deren Mischungen mit einem Brechungsindex von 1,50 bis 2,00 und mit einer durchschnittlichen Teilchengrösse von 0,1 bis 5,0µm.

Die Bestandteile A und B können aber auch einzeln für sich als Dentalglaskomponente verwendet werden.

Der anorganische Füllstoff (A) enthält als Oxid eines Metalls der Gruppen I, II, , IV, V, XII, XIII und XIV des Periodensystems vorzugsweise Strontium- , Aluminium-, Zink-, Titan-, Lanthan- und/oder Zirkonoxid. Die durchschnittliche Primärteilchengrösse liegt vorzugsweise im Bereichen 0,15 bis 2,0µm, und der Brechungsindex des anorganischen Füllstoffs (A) vorzugsweise zwischen 1,57 und 1,64. Ein besonders bevorzugter Wert ist 1,60 ± 0,02. Der Füllstoff des Typs (A) kann auch gesintert als Mischung von Agglomeraten mit einer durchschnittlichen Teilchengrösse von 0,5 - 2,0 µm vorliegen.

Die durchschnittlichen Primärteilchengrösse des anorganischen Füllstoffs (B) liegt vorzugsweise zwischen 0,1 und 5,0 µm und besonders bevorzugt zwischen 0,5 und 2,0 µm, während der Brechungsindex Werte vorzugsweise zwischen 1,57 und 1,64 aufweisen soll. Es können auch Füllstoffgemische eingesetzt werden. Bevorzugt werden erfindungsgemäss Ba-Silikatgläser mit einer mittleren Korngrösse im Bereich von 0,4 bis 2,0 µm, sowie Li/Al-Silikatgläser mit einer mittleren Korngrösse von 0,4 bis 2,0 µm verwendet.

Die Oberfläche der Dentalglaspartikel kann mit einem, vernetzbare Doppelbindungen aufweisenden Haftvermittler, vorzugsweise Gamma-methacryloxypropyltrimethoxysilan versehen sein.

Vorteilhafterweise weisen die Dentalglaspartikel einen Gehalt an Siliziumoxid von weniger als 35 Gew-%, vorzugsweise von weniger als 30 Gew-% auf. Gegenüber Gläsern mit einem höheren Siliziumoxidanteil ergibt sich der Vorteil, dass mehr ZrO₂ oder ZnO, u.s.w. zugegeben werden kann, welche den Brechungsindex erhöhen.

Die nanoskaligen anorganischen Feststoffteilchen, vorzugsweise das Zirkonoxid, weisen vorteilhafterweise eine spezifische Oberfläche im Bereich von 110 - 250 m²/g auf.

Die Dentalglaspartikel weisen vorteilhafterweise eine spezifische Oberfläche im Bereich von 12 -15 m²/g (für eine Partikelgrösse von 0,7 µm), 7-8 m²/g (für eine Partikelgrösse von 1,0 µm) und von 1-2 m²/g (für eine Partikelgrösse von 5,0 µm) auf.

Die Dichte der Dentalglaspartikel liegt vorzugsweise im Bereich von 2,5 - 4,7 g/cm³; (im speziellen bei 3,42). Die nanoskaligen anorganischen Feststoffteilchen weisen vorzugsweise eine Dichte von 3,0 - 6,5 g/cm³ auf (im speziellen 5,4).

Der Brechungsindex des ausgehärteten Dentalmaterials liegt im Bereich von 1,56 - 1,70, vorzugsweise von 1,58 - 1,64.

Die Erfindung und Weiterbildungen der Erfindung werden im folgenden anhand von mehreren Ausführungsbeispielen näher erläutert.

### Beispiel 1 (Herstellung einer lichthärtenden Dentalharzmischung)

### - nicht erfindungsgemäß

Es wurde eine Dentalharzmischung aus folgenden Komponenten:
30.0 g Urethan-dimethacrylat
55,0 g Bis-GMA
15,0 g Hexandioldimethacrylat
0.20 g Campherchinon
0.20 g Ethyl-p-dimethylaminobenzoat

in einem herkömmlichen Planetenmischer hergestellt. Die erhaltene homogene Mischung wies einen Brechungsindex n_{D}²⁰ = 1,5140 auf.

### Beispiel 2 (Herstellung einer zirkonoxidhaltigen Dentalharzmischung)

Zu 50,0 g der in Beispiel 1 erhaltenen Dentalharzmischung wurden 160,0 g eines zirkonoxidhaltigen Soles in Ethanol (40%ig) beigemischt. Die Grösse der Zirkonoxidpartikel betrug 4 - 20 nm. Die Dentalharzmischung wurde zu diesem Zweck im Sol durch Rühren mit einem Magnetrührer oder durch Schütteln vollständig aufgelöst. Die erhaltene zirkonoxidhaltige Dentalharzmischung wurde mit Hilfe eines Rotationsverdampfers unter abgedunkelten Bedingungen von leicht flüchtigen Bestandteilen befreit und 30 min. bei Raumtemperatur im Vakuum getrocknet.

Der Brechungsindex der so erhaltenen Mischung betrug n_{D}²⁰ = 1,5880.

### Beispiel 3 (Herstellung einer zirkonoxidhaltigen Dentalharzmischung)

Zu 50,0 g der in Beispiel 1 erhaltenen Dentalharzmischung wurden 180 g eines zirkonoxidhaltigen Soles in Ethanol (40 %ig) - analog zum Beispiel 2 - beigemischt. Die erhaltene zirkonoxidhaltige Dentalharzmischung wurde mit Hilfe eines Rotationsverdampfers unter abgedunkelten Bedingungen von leicht flüchtigen Bestandteilen befreit und 30 min. bei Raumtemperatur im Vakuum getrocknet. Der Brechungsindex der so erhaltenen Mischung betrug n_{D}²⁰ = 1,6050

### Beispiel 4 (Herstellung eines Dental-Komposits)

Zu 85,0 g der in Beispiel 3 erhaltenen Mischung wurden 130,0 g silanisiertes Glaspulver beigemischt. Das Glaspulver wies folgende Eigenschaften auf:

| | |
|---|---|
| Mittlere Partikelgrösse | : 0,7 µm |
| Brechungsindex n_{D}²⁰ | : 1,6000 |
| Dichte (p) | : 3,42 |
| Zusammensetzung | : 30 % SiO₂ / 25 % SrO /10 % ZnO /10 % ZrO₂ / |
| Rest: | B₂O₃ /Al₂O₃/ La₂O₃ / CaO / Na₂O |

Diese Mischung wurde in einem herkömmlichen Planetenmischer zu einer homogenen Paste verarbeitet und wies einen Brechungsindex n_{D}²⁰ = 1,6060 auf.

Eine Probe der Paste wurde mit einem herkömmlichen Lichtpolymerisationsgerät (Spektra 2000; Schütz-Dental) 9 Minuten lang ausgehärtet. Das erhaltene ausgehärtete Komposit wies folgende Eigenschaften auf:
Brechungsindex n_{D}²⁰ = 1,6060
Vicker's Härte, Oberfläche: 44,5
Vicker's Härte, 2 mm Tiefe: 16,9

### Beispiel 5 (Herstellung eines Dental-Komposits)

Zu 85,0 g der in Beispiel 3 erhaltenen Mischung wurden 130,0 g silanisiertes Glaspulver beigemischt. Das Glaspulver wies folgende Eigenschaften auf:

| | |
|---|---|
| Mittlere Partikelgrösse | 1,0 µm |
| Brechungsindex n_{D}²⁰ | : 1,6000 |
| Dichte (p) | : 3,42 |
| Zusammensetzung | : 30 % SiO₂ / 25 % SrO / 10 % ZnO /10 % ZrO₂/ |
| | Rest: B₂O₃ /Al₂O₃/ La₂O₃/CaO / Na₂O |

Diese Mischung wurde in einem herkömmlichen Planetenmischer zu einer homogenen Paste verarbeitet und wies einen Brechungsindex n_{D}²⁰ = 1,6050 auf. Eine Probe der Paste wurde mit einem herkömmlichen Lichtpolymerisationsgerät (Spektrum 2000; Schütz-Dental) 9 Minuten lang ausgehärtet. Das erhaltene ausgehärtete Komposit wies folgende Eigenschaften auf:
Brechungsindex n_{D}²⁰ = 1,6050
Vicker's Härte, Oberfläche: 49,9
Vicker's Härte, 2 mm Tiefe: 34,1

### Beispiel 6 (Herstellung einer Dentalharzmischung)

### - nicht erfindungsgemäß

Es wurde eine Dentalharzmischung aus folgenden Komponenten:
40 g Bis-GMA
88 g Urethan-dimethacrylat
32 g Hexandioldimethacrylat
0,32 g Campherchinon
0,32 g Ethyl-p-dimethylaminobenzoat

in einem herkömmlichen Planetenmischer hergestellt. Die erhaltene homogene Mischung wies einen Brechungsindex n_{D}²⁰ = 1,4960 auf.

### Beispiele 7 - 16 (Herstellung der zirkonoxidhaltigen Dentalharzmischungen)

Die Tabelle zeigt den Brechungsindex der Mischung aus Dentalharz gemäss Beispiel 6 und einem zirkonoxidhaltigen Sol in Abhängigkeit des Zirkonoxidgehaltes:

| Beispiel Nr. | Gehalt in Gramm an Monomermischung aus Beispiel 6 | Gehalt in Gramm an Zirkonoxid Sol 40%ig | Brechungsindex n_{D}²⁰ |
|---|---|---|---|
| 7 | 160 | 225 | 1,5440 |
| 8 | 160 | 320 | 1,5590 |
| 9 | 160 | 384 | 1,5670 |
| 10 | 160 | 417 | 1,5710 |
| 11 | 160 | 448 | 1.5825 |
| 12 | 160 | 480 | 1.5860 |
| 13 | 160 | 512 | 1.5950 |
| 14 | 160 | 622 | 1.6025 |
| 15 | 160 | 800 | 1.6240 |
| 16 | 160 | 874 | 1.6350 |

### Beispiel 17 (Herstellung eines Dental-Komposits)

Zu 85,0 g der in Beispiel 13 erhaltenen Mischung wurden 130,0 g silanisiertes Glaspulver beigemischt. Das Glaspulver wies folgende Eigenschaften auf:

| | |
|---|---|
| Partikelgrösse | : 1,0 µm |
| Brechungsindex n_{D}²⁰ | : 1,6000 |
| Dichte (p) | : 3,42 |
| Zusammensetzung | : 30 % SiO₂ / 25 % SrO / 10 % ZnO / 5 % La₂O₃/ 5 % Al₂O₃ / |
| | Rest: B₂O₃ / CaO / Na₂O / ZrO₂ |

Diese Mischung wurde in einem herkömmlichen Planetenmischer zu einer homogenen Paste verarbeitet. Eine Probe der Paste wurde mit einem herkömmlichen Lichtpolymerisationsgerät (Spektra 2000; Schütz-Dental) 9 Minuten lang ausgehärtet.

Das erhaltene ausgehärtete Komposit wies folgende Eigenschaften auf:
n_{D}²⁰ = 1,5955
Vicker's Härte, Oberfläche: 52,7
Vicker's Härte, 2 mm Tiefe: 40,1

### Beispiel 18 (Herstellung eines Dental-Komposits)

Zu 85,0 g der in Beispiel 13 erhaltenen Mischung wurden 130,0 g silanisiertes Glaspulver beigemischt. Das Glaspulver wies folgende Eigenschaften auf:

| | |
|---|---|
| Mittlere Partikelgrösse | 0,7 µm |
| Brechungsindex n_{D}²⁰ | : 1,6000 |
| Dichte (p) | : 3,42 |
| Zusammensetzung | : 30 % SiO₂ / 25 % SrO /10 % ZnO /10 % ZrO₂/ |
| | Rest: B₂O₃/Al₂O₃/La₂O₃/CaO/Na₂O |

Diese Mischung wurde in einem herkömmlichen Planetenmischer zu einer homogenen Paste verarbeitet. Eine Probe der Paste wurde mit einem herkömmlichen Lichtpolymerisationsgerät (Spektra 2000; Schütz-Dental) 9 Minuten lang ausgehärtet. Das erhaltene ausgehärtete Komposit wies folgende Eigenschaften auf:
n_{D}²⁰ = 1,5945
Vicker's Härte, Oberfläche: 50,5
Vicker's Härte, 2 mm Tiefe: 35,5

### Beispiel 19 (Herstellung einer zirkonoxidhaltigen Dentalharzmischung)

Es wurde eine Dentalharzmischung aus folgenden Komponenten:
50,0 g Bis-GMA (mit dem Brechungsindex n_{D}²⁰ = 1,5503
100,0 g 40 %iges zirkonoxidhaltiges Sol in Ethanol
0,05 g Campherchinon
0,05 g Ethyl-p-dimethylaminobenzoat

in einem herkömmlichen Planetenmischer hergestellt (analog zu Beispiel 2). Die erhaltene homogene Mischung wies einen Brechungsindex n_{D}²⁰ =1,5920 auf.

### Beispiel 20 (Herstellung eines Dental-Komposits)

100 g der in Beispiel 19 erhaltenen Mischung wurden mit 130 g silanisiertem Glaspulver vermischt. Das Glaspulver wies folgende Eigenschaften auf:

| | |
|---|---|
| Mittlere Partikelgrösse | 0,7 µm |
| Brechungsindex n_{D}²⁰ | : 1,6000 |
| Dichte (p) | : 3,42 |
| Zusammensetzung | : 30 % SiO₂ / 25 % SrO /10 % ZnO /10 % ZrO₂/ |
| | Rest: B₂O₃ /Al₂O₃/La₂O₃/CaO/Na₂O |

Diese Mischung wurde in einem herkömmlichen Planetenmischer zu einer homogenen Paste verarbeitet. Eine Probe der Paste wurde mit einem herkömmlichen Lichtpolymerisationsgerät (Spektra 2000; Schütz-Dental) 9 Minuten lang ausgehärtet.

Das erhaltene ausgehärtete Komposit wies folgende Eigenschaften auf:
n_{D}²⁰ = 1,6010
Vicker's Härte, Oberfläche: 35,8
Vicker's Härte, 2 mm Tiefe: 19,5

### Beispiel 21 (Herstellung eines Dental-Komposits)

100 g der in Beispiel 19 erhaltenen Mischung wurden mit 130 g silanisiertem Glaspulver vermischt. Das Glaspulver wies folgende Eigenschaften auf:

| | |
|---|---|
| Mittlere Partikelgrösse | 5,0 µm |
| Brechungsindex n_{D}²⁰ | : 1,6000 |
| Dichte (p) | : 3,42 |
| Zusammensetzung | : 30 % SiO₂ / 25 % SrO/10 % ZnO / 5 % La₂O₃/ 5 % Al₂O₃ / |
| | Rest: B₂O₃ / CaO / Na₂O /ZrO₂ |

Diese Mischung wurde in einem herkömmlichen Planetenmischer zu einer homogenen Paste verarbeitet. Eine Probe der Paste wurde mit einem herkömmlichen Lichtpolymerisationsgerät (Spektra 2000; Schütz-Dental) 9 Minuten lang ausgehärtet.

Das erhaltene ausgehärtete Komposit wies folgende Eigenschaften auf:
n_{D}²⁰ = 1,6060
Vicker's Härte, Oberfläche: 27,4
Vicker's Härte, 2 mm Tiefe: 18,2

### Beispiel 22

### (Herstellung einer autopolymerisierenden 2-Komponenten Dentalharzmischung)

### Grundharzmischung:

30.0 g Urethan-dimethacrylat
55,0 g Bis-GMA
15,0 g Hexandioldimethacrylat

### Zirkonoxidhaltige Dentalharzmischung:

50,0 g der oben angegebenen Grundharzmischung
180,0 g eines zirkonoxidhaltigen Soles in Ethanol (40%ig)

### Komponente A:

85.0 g der obigen zirkonoxidhaltige Dentalharzmischung
0,27g N, N-Bis-(2)-Hydroxyethyl)-p-toluidin
130.0 g Glaspulver (mittlere Partikelgrösse 0.7 µm)

### Komponente B:

85.0 g der obigen zirkonoxidhaltige Dentalharzmischung
12,5 g Dibenzoylperoxid (50%iges Pulver in Phthalat)
130.0 g Glaspulver (0.7 µm)

Gleiche Mengen an Komponente A und B wurden mit einem Spatel während 30 Sekunden vermischt und diese Masse zur Restauration einer Schmelzläsion verwendet. Die ausgehärtete Masse auf dem Zahn ergab eine hohe Transluzenz und eine dem natürlichen Schmelz weitgehend entsprechendes Aussehen.

Das erfindungsgemässe Dentalmaterial kann in fliessfähiger oder auch knetbarer Form hergestellt werden. Alle ausgehärteten Dentalkompositmaterialien wiesen bei 1 mm Schichtdicke eine hervorragende Transparenz auf.

## Patentansprüche

1. Dentalmaterial, welches ein polymerisierbares Dentalharz mit dem Brechungsindex n₁, nanoskalige anorganische Feststoffteilchen mit dem Brechungsindex n₂, Dentalglaspartikel mit dem Brechungsindex n₃ sowie Hilfsstoffe für die Autopolymerisation oder Lichthärtung des Dentalharzes enthält,
**dadurch gekennzeichnet, dass**
A) das Dentalmaterial insgesamt einen Brechungsindex n₄ aufweist, welcher im Bereich von 1,56 - 1,70 liegt;
B) der Brechungsindex n₅ des ausgehärteten Dentalmaterials im Bereich von 1,56-1,70 liegt; und
C) die nanoskaligen anorganischen Feststoffteilchen einen Durchmesser kleiner als 100 nm aufweisen.

2. Dentalmaterial nach Anspruch 1, **dadurch gekennzeichnet, dass** der Brechungsindex n₄ grösser als 1,59, vorzugsweise grösser als 1,60 ist.

3. Dentalmaterial nach Anspruch 1 oder 2, **dadurch gekennzeichnet, dass** der Brechungsindex n₄ kleiner als 1,64, vorzugsweise kleiner als 1,62 ist.

4. Dentalmaterial, nach einem der Ansprüche 1 bis 3, **dadurch gekennzeichnet, dass** das allein mit den nanoskaligen anorganischen Feststoffteilchen vermischte Dentalharz einen Brechungsindex n₁₂ aufweist, welcher um höchstens 5 %, vorzugsweise höchstens 2 % vom Brechungsindex n₃ der Dentalglaspartikel abweicht.

5. Dentalmaterial nach Anspruch 4, **dadurch gekennzeichnet, dass** der Brechungsindex n₁₂ höchstens um 1,0 %, vorzugsweise höchstens um 0,2 % vom Brechungsindex n₃ abweicht.

6. Dentalmaterial nach Anspruch 4 oder 5, **dadurch gekennzeichnet, dass** der Brechungsindex n₁₂ im Bereich von 1,56 bis 1,65, vorzugsweise von 1,59 bis 1,62 liegt.

7. Dentalmaterial nach einem der Ansprüche 4 bis 6, **dadurch gekennzeichnet, dass** der Brechungsindex n₃ im Bereich von 1,58 bis 1,65, vorzugsweise von 1,59 bis 1,62 liegt.

8. Dentalmaterial nach einem der Ansprüche 1 bis 7, **dadurch gekennzeichnet, dass** der Brechungsindex n₁ des Dentalharzes grösser als 1,54 ist.

9. Dentalmaterial nach einem der Ansprüche 1 bis 8, **dadurch gekennzeichnet, dass** das Dentalharz ein Bis-Methacrylat, vorzugsweise Bis-GMA oder ein ethoxyliertes Bisphenol-A-dimethacrylat ist.

10. Dentalmaterial nach einem der Ansprüche 1 bis 8, **dadurch gekennzeichnet, dass** das Dentalharz ein methacryl-substituiertes Poly-Di-Phenyl-silikon oder Diphenylsilan ist.

11. Dentalmaterial nach einem der Ansprüche 1 bis 8, **dadurch gekennzeichnet, dass** das Dentalharz aus der Gruppe: Poly(pentabromophenyl-methacrylate, Poly(pentabromophenyl-acrylate), Poly(pentabromobenzyl-methacrylate), Poly(pentabromobenzyl-acrylate, Poly(2,4,6-tribromophenyl-methacrylate), Poly(vinylphenylsulfide), Poly(1-naphthylmethacrylate), Poly(2-vinylthiophene, Poly(2,6-dichlorostyrene), Poly(N-vinylphthalimide), Poly(2-chlorostyrene), Poly(pentachlorophenyl methacrylate) ausgewählt ist.

12. Dentalmaterial nach einem der Ansprüche 1 bis 11, **dadurch gekennzeichnet, dass** die nanoskaligen anorganischen Feststoffteilchen auf der Basis von Metalloxiden vorzugsweise Titandioxid, Zinkoxid, Zirkonoxid oder Aluminiumoxid, sowie Mischungen davon ausgewählt sind.

13. Dentalmaterial nach einem der Ansprüche 1 bis 12, **dadurch gekennzeichnet, dass** das Gewichtsverhältnis zwischen dem Dentalharz und den nanoskaligen anorganischen Feststoffteilchen im Bereich von 1,0 bis 2,5 liegt.

14. Dentalmaterial nach einem der Ansprüche 1 bis 13, **dadurch gekennzeichnet, dass** die Oberfläche der nanoskaligen anorganischen Feststoffteilchen mit (i) einer organischen Säure, vorzugsweise einer Karbonsäure oder (ii) einem methacrylatsubstituierten Silan beschichtet ist.

15. Dentalmaterial nach einem der Ansprüche 1 bis 14, **dadurch gekennzeichnet, dass** die Dentalglaspartikel einen maximalen mittleren Durchmesser von 5 µm, vorzugsweise maximal 0,7 µm aufweisen.

16. Dentalmaterial nach einem der Ansprüche 1 bis 15, **dadurch gekennzeichnet, dass** die Oberfläche der Dentalglaspartikel mit einem, vernetzbare Doppelbindungen aufweisenden Haftvermittler, vorzugsweise Gamma-methacryloxypropyltrimethoxysilan versehen ist.

17. Dentalmaterial nach einem der Ansprüche 1 bis 16, **dadurch gekennzeichnet, dass** die Dentalglaspartikel einen Gehalt an Siliziumoxid von weniger als 35 Gew-%, vorzugsweise von weniger als 30 Gew-% aufweisen

18. Dentalmaterial nach einem der Ansprüche 12 bis 16, **dadurch gekennzeichnet, dass** die nanoskaligen anorganischen Feststoffteilchen, vorzugsweise das Zirkonoxid eine spezifische Oberfläche im Bereich von 110 - 250 m²/g aufweisen.

19. Dentalmaterial nach einem der Ansprüche 1 bis 18, **dadurch gekennzeichnet, dass** die Dentalglaspartikel eine spezifische Oberfläche im Bereich von 12 -15 m²/g (für eine Partikelgrösse von 0,7 µm), 7-8 m²/g (für eine Partikelgrösse von 1,0 µm) und von 1-2 m²/g (für eine Partikelgrösse von 5,0 µm) aufweisen.

20. Dentalmaterial nach einem der Ansprüche 1 bis 19, **dadurch gekennzeichnet, dass** die Dentalglaspartikel eine Dichte von 2,5 - 4,7 g/cm³ aufweisen.

21. Dentalmaterial nach einem der Ansprüche 1 bis 20, **dadurch gekennzeichnet, dass** die nanoskaligen anorganischen Feststoffteilchen eine Dichte von 3,0 - 6,5 g/cm³ aufweisen.

22. Dentalmaterial nach einem der Ansprüche 1 bis 21, **dadurch gekennzeichnet, dass** der Brechungsindex n₅ des ausgehärteten Dentalmaterials im Bereich von 1,58- 1,64 liegt.

23. Dentalmaterial nach einem der Ansprüche 1 bis 22, **dadurch gekennzeichnet, dass** es nach erfolgter Aushärtung in einer Schichtdicke von 1 mm transparent ist.

24. Dentalmaterial nach einem der Ansprüche 1 bis 23, **dadurch gekennzeichnet, dass** das Dentalmaterial insgesamt transparent ist.

## Claims

1. Dental material containing a polymerizable dental resin with the index of refraction n₁, nanoscale inorganic solid particles with the index of refraction n₂, dental glass particles with the index of refraction n₃, and also adjuvants for the autopolymerization or light-curing of the dental resin,
**characterized in that**
A) the dental material has an overall index of refraction n₄, which is in the range of 1.56 - 1.70;
B) the index of refraction n₅ of the cured dental material is in the range of 1.56 - 1.70; and
C) the nanoscale inorganic solid particles have a diameter less than 100 nm.

2. Dental material according to claim 1, **characterized in that** the index of refraction n₄ is greater than 1.59, preferably greater than 1.60.

3. Dental material according to claim 1 or claim 2, **characterized in that** the index of refraction n₄ is smaller than 1.64, preferably smaller than 1.62.

4. Dental material according to one of claims 1 through 3, **characterized in that** the dental resin mixed with just the nanoscale inorganic solid particles has an index of refraction n₁₂, which deviates from the index of refraction n₃ of the dental glass particles by at most 5%, preferably by at most 2%.

5. Dental material according to claim 4, **characterized in that** the index of refraction n₁₂ deviates from the index of refraction n₃ by at most 1.0%, preferably by at most 0.2%.

6. Dental material according to claim 4 or claim 5, **characterized in that** the index of refraction n₁₂ is in the range of 1.56 to 1.65, preferably in the range of 1.59 to 1.62.

7. Dental material according to one of claims 4 through 6, **characterized in that** the index of refraction n₃ is in the range of 1.58 to 1.65, preferably in the range of 1.59 to 1.62.

8. Dental material according to one of claims 1 through 7, **characterized in that** the index of refraction n₁ of the dental resin is greater than 1.54.

9. Dental material according to one of claims 1 through 8, **characterized in that** the dental resin is a bis-methacrylate, preferably bis-GMA, or a bisphenol-A-ethoxylate dimethacrylate.

10. Dental material according to one of claims 1 through 8, **characterized in that** the dental resin is a methacryl-substituted poly-di-phenyl silicone or diphenyl silane.

11. Dental material according to one of claims 1 through 8, **characterized in that** the dental resin is chosen from the group comprising: poly(pentabromophenyl-methacrylates), poly(pentabromophenyl-acrylates), poly(pentabromobenzyl-methacrylates), poly(penta-bromobenzyl-acrylates, poly(2,4,6-tribromophenyl-methacrylates), poly(vinylphenyl sulfides), poly(1-naphthylmethacrylates), poly(2-vinylthiophenes, poly(2,6-di-chlorostyrenes), poly(n-vinylphthalimides), poly(2-chlorostyrenes), poly(pentachlorophenyl methacrylates).

12. Dental material according to one of claims 1 through 11, **characterized in that** the nanoscale inorganic solid particles are chosen to be metal oxides, preferably titanium dioxide, zinc oxide, zirconium oxide, or aluminum oxide, as well as mixtures thereof.

13. Dental material according to one of claims 1 through 12, **characterized in that** the weight ratio of the dental resin to the nanoscale inorganic particles is in the range of 1.0 to 2.5.

14. Dental material according to one of claims 1 through 13, **characterized in that** the surface of the nanoscale inorganic particles is coated with (i) an organic acid, preferably a carboxylic acid or (ii) a methacryl-substituted silane.

15. Dental material according to one of claims 1 through 14, **characterized in that** the dental glass particles have a maximum mean diameter of 5 µm, preferably at most 0.7 µm.

16. Dental material according to one of claims 1 through 15, **characterized in that** the surface of the dental glass particles is provided with an adhesive means comprising crosslinking double bonds, preferably gamma-methacryloxypropyltrimethoxysilane.

17. Dental material according to one of claims 1 through 16, **characterized in that** the dental glass particles contain less than 35 wt %, preferably less than 30 wt %, of silicon oxide.

18. Dental material according to one of claims 12 through 16, **characterized in that** the nanoscale inorganic solid particles, preferably the zirconium oxide, have a specific surface area in the range of 110-250 m²/g.

19. Dental material according to one of claims 1 through 18, **characterized in that** the dental glass particles have a specific surface area in the range of 12-15 m²/g (for a particle size of 0.7 µm), 7-8 m²/g (for a particle size of 1.0 µm), and 1-2 m²/g (for a particle size of 5.0 µm).

20. Dental material according to one of claims 1 through 19, **characterized in that** the dental glass particles have a density of 2.5-4.7 g/cm³.

21. Dental material according to one of claims 1 through 20, **characterized in that** the nanoscale inorganic solid particles have a density of 3.0-6.5 g/cm³.

22. Dental material according to one of claims 1 through 21, **characterized in that** the index of refraction n₅ of the cured dental material is in the range of 1.58-1.64.

23. Dental material according to one of claims 1 through 22, **characterized in that** after curing, it has a transparent layer with a thickness of 1 mm.

24. Dental material according to one of claims 1 through 23, **characterized in that** said dental material is transparent overall.

## Revendications

1. Matériel dentaire, lequel contient une résine dentaire polymérisable doté d'un indice de réfraction n₁, des particules solides inorganiques à l'échelle nanométrique doté d'un indice de réfraction n₂, des particules de verre dentaire doté d'un indice de réfraction n₃, ainsi que des excipients pour l'autopolymérisation ou le photodurcissage de la résine dentaire, **caractérisé en ce que**
A) le matériel dentaire présente dans l'ensemble un indice de réfraction n₄, lequel se situe dans une plage comprise entre 1,56 et 1,70 ;
B) l'indice de réfraction n₅ du matériel dentaire durci se situe dans une plage comprise entre 1,56 et 1,70 ; et
C) les particules solides inorganiques à l'échelle nanométrique présentent un diamètre inférieur à 100 nm.

2. Matériel dentaire selon la revendication 1, **caractérisé en ce que** l'indice de réfraction n₄ est supérieur à 1,59, de préférence supérieur à 1,60.

3. Matériel dentaire selon la revendication 1 ou 2, **caractérisé en ce que** l'indice de réfraction n₄ est inférieur à 1,64, de préférence inférieur à 1,62.

4. Matériel dentaire, selon l'une des revendications 1 à 3, **caractérisé en ce que** la résine dentaire, mélangée seule avec les particules solides inorganiques à l'échelle nanométrique, présente un indice de réfraction n₁₂, lequel diffère d'au maximum 5%, de préférence d'au maximum 2%, de l'indice de réfraction n₃ des particules de verre dentaire.

5. Matériel dentaire selon la revendication 4, **caractérisé en ce que** l'indice de réfraction n₁₂ diffère d'au maximum 1,0%, de préférence d'au maximum 0,2%, de l'indice de réfraction n₃.

6. Matériel dentaire selon la revendication 4 ou 5, **caractérisé en ce que** l'indice de réfraction n₁₂ se situe dans une plage comprise entre 1,56 et 1,65, de préférence entre 1,59 et 1,62.

7. Matériel dentaire selon l'une des revendications 4 à 6, **caractérisé en ce que** l'indice de réfraction n₃ se trouve se situe dans une plage comprise entre 1,58 à 1,65, de préférence de 1,59 à 1,62.

8. Matériel dentaire selon l'une des revendications 1 à 7, **caractérisé en ce que** l'indice de réfraction n₁ de la résine dentaire est supérieur à 1,54.

9. Matériel dentaire selon l'une des revendications 1 à 8, **caractérisé en ce que** la résine dentaire est un bisméthacrylate, de préférence un bis-GMA ou un diméthacrylate de bisphénol-A éthoxylé.

10. Matériel dentaire selon l'une des revendications 1 à 8, **caractérisé en ce que** la résine dentaire est un silicone polydiphényle à substitution méthacrylique ou un diphénylsilane.

11. Matériel dentaire selon l'une des revendications 1 à 8, **caractérisé en ce que** la résine dentaire est sélectionnée dans le groupe : poly(méthacrylate de pentabromophényle), poly(acrylate de pentabromophényle), poly(méthacrylate de pentabromobenzyle), poly(acrylate de pentabromobenzyle), poly(méthacrylate de 2,4,6-tribromophényle), poly(sulfure de vinyl-phényle), poly(méthacrylate de 1-naphtyle), poly(2-vinylthiophène), poly(2,6-dichlorostyrène), poly(N-vinylphtalimide), poly(2-chlorostyrène), poly(méthacrylate de pentachlorophényle).

12. Matériel dentaire selon l'une des revendications 1 à 11, **caractérisé en ce que** les particules solides inorganiques à l'échelle nanométrique sont sélectionnées sur la base d'oxydes de métal, de préférence de dioxyde de titane, d'oxyde de zinc, d'oxyde de zirconium ou d'oxyde d'aluminium, ainsi que de mélanges de ceux-ci.

13. Matériel dentaire selon l'une des revendications 1 à 12, **caractérisé en ce que** le rapport pondéral entre la résine dentaire et les particules solides inorganiques à l'échelle nanométrique se situe dans une plage comprise entre 1,0 et 2,5.

14. Matériel dentaire selon l'une des revendications 1 à 13, **caractérisé en ce que** la surface des particules solides inorganiques à l'échelle nanométrique est revêtue (i) d'un acide organique, de préférence d'un acide carboxylique, ou (ii) d'un silane à substitution méthacrylique.

15. Matériel dentaire selon l'une des revendications 1 à 14, **caractérisé en ce que** les particules de verre dentaire présentent un diamètre moyen maximal de 5 µm, de préférence d'au plus 0,7 µm.

16. Matériel dentaire selon l'une des revendications 1 à 15, **caractérisé en ce que** la surface des particules de verre dentaire est pourvue d'un agent d'adhérence présentant des doubles liaisons réticulables, de préférence gamma-méthacryloxypropyltriméthoxysilane.

17. Matériel dentaire selon l'une des revendications 1 à 16, **caractérisé en ce que** les particules de verre dentaire présentent une teneur en oxyde de silicium inférieure à 35 % en poids, de préférence inférieure à 30 % en poids.

18. Matériel dentaire selon l'une des revendications 12 à 16, **caractérisé en ce que** les particules solides inorganiques à l'échelle nanométrique, de préférence l'oxyde de zirconium, présentent une surface spécifique se situant dans une plage comprise entre 110 et 250 m²/g.

19. Matériel dentaire selon l'une des revendications 1 à 18, **caractérisé en ce que** les particules de verre dentaire présentent une surface spécifique se situant dans une plage de 12-15 m²/g (pour une taille de particules de 0,7 µm), de 7- 8 m²/g (pour une taille de particules de 1,0 µm) et de 1-2 m²/g (pour une taille de particules de 5,0 µm).

20. Matériel dentaire selon l'une des revendications 1 à 19, **caractérisé en ce que** les particules de verre dentaire présentent une densité de 2,5 à 4,7 g/cm³.

21. Matériel dentaire selon l'une des revendications 1 à 20, **caractérisé en ce que** les particules solides inorganiques à l'échelle nanométrique présentent une densité de 3,0 à 6,5 g/cm³.

22. Matériel dentaire selon l'une des revendications 1 à 21, **caractérisé en ce que** l'indice de réfraction n₅ du matériel dentaire durci se situe dans une plage comprise entre 1,58 et 1,64.

23. Matériel dentaire selon l'une des revendications 1 à 22, **caractérisé en ce que**, à l'issue du durcissement, le matériel est transparent sur une épaisseur de couche de 1 mm.

24. Matériel dentaire selon l'une des revendications 1 à 23, **caractérisé en ce que** le matériel dentaire est transparent dans sa totalité.
